# EUROPEAN PATENT APPLICATION

(11) **EP 3 896 449 A1**
(43) Date of publication of application: **20.10.2021**
(21) Application number: 20305372.3
(22) Date of filing: 17.04.2020
(51) Int. Cl.: G01N 33/68

(54) **METHOD OF PROGNOSIS OF LEFT VENTRICULAR REMODELING**

(71) Applicant: Assistance Publique Hôpitaux de Paris, 75004 Paris (FR); Institut National de la Santé et de la Recherche Médicale (INSERM), 75013 Paris (FR); Université de Paris, 75006 Paris (FR)
(72) Inventor: VODOVAR, Nicolas, 92120 MONTROUGE (FR); LOGEART, Damien, 91260 JUVISY SUR ORGE (FR); LAUNAY, Jean-Marie, 95100 Argenteuil (FR)
(74) Representative: Flesselles, Bruno F.G.

(57) **Abstract**

The present invention relates to PrPc as a new serum marker useful by itself for diagnosis or prognosis of the occurrence of deleterious left ventricular remodeling in patients having suffered myocardial infarction.

## Description

The present invention relates to PrPc as a new serum marker useful by itself for diagnosis or prognosis of the occurrence of deleterious left ventricular remodeling in patients having suffered myocardial infarction.

Myocardial infarction (MI) occurs when blood flow decreases or stops to a part of the heart, causing damage to the heart muscle. Most MIs occur due to coronary artery disease.

Heart failure may develop as a long-term consequence, with an impaired ability of heart muscle to pump, scarring, and increase in the size of the existing muscle. Remodeling of the left ventricle myocardium develops in about 10% of MI and is itself a risk factor for future hear problems such as heart failure, ventricular arrhythmia and the development of clots.

Indeed, following injury of the left ventricle, it may enlarge, its general shape may change, with the muscular wall of the ventricle becoming thinner. This will result in a degraded heart function.

Early after a heart attack, some remodeling can be beneficial as it can compensate for the damage that has occurred in the ventricle. Remodeling becomes deleterious if it continues, as the changes in the size and shape of the ventricle lead to cardiac function deterioration.

Remodeling of the heart is generally evaluated by performing an echocardiogram. The size and function of the atria and ventricles can be characterized using this test.

Medications may attenuate remodeling. In particular, angiotensin-converting enzyme (ACE) inhibitors have been shown to decrease remodeling in animal models or transmural infarction and chronic pressure overload. Inhibition of aldosterone-related effects by Mineralo Receptor Antagonists (MRA, such as Spironolactone or Eplerenone) is also beneficial to decrease remodeling. The beta blockers of the latest generation may also reverse the remodeling process by reducing left ventricular volumes and improving systolic function.

In sum, treatments indicated to prevent remodeling are beta-blockers (such as bisoprolol, carvedilol, or sustained-release metoprolol), ACE inhibitors (such as benazepril, zofenopril, perindopril, trandolapril, captopril, enalapril, lisinopril, or ramipril) or ARB (Angiotensin II Receptor Blockers, such as azilsartan, candesartan, eprosartan, irbesartan, losartan, olmesartan, telmisartan or valsartan) and MRA.

Beta-blockers are prescribed to all patients for a minimum of 6-12 months, unless there is a problem of intolerance. ACE inhibitors or ARB are prescribed in many patients with MI. Recommendations to use these molecules are formal if LVEF (Left Ventricular Ejection Fraction, as measured by echography) is lower than 40% or if there was heart failure during the initial phase. Some physician recommend to use these molecules for all patients with MDI. MRA is indicated (and prescribed) if LVEF is lower than 35-40% or if heart failure was observed.

Currently, a new class (ARNI, ARB + neprilysin inhibitor) is being tested in MI with LVEF <35% or clinical heart failure, with results that will most likely be positive (it already works very well in chronic heart failure with LVEF < 40%).

All of these treatments have the main objective of reducing future remodeling.

There is thus a need to develop new diagnosis tests, reliable, easy to perform, that are able to detect the presence of left ventricular remodeling, after myocardial infarction or to predict at an early stage whether such remodeling will occur or determine a risk that remodeling will occur. This will make it possible to provide appropriate treatments and follow-up to the patients to reduce the remodeling process.

The inventors demonstrated that dosing the amount of a PrPC in a biological sample (blood or plasma) of a patient makes it possible to detect presence of left ventricular remodeling if such dosing is performed about 6 months after myocardial infarction, or to predict the risk of LVR within 6 months if such dosing is performed shortly (within one week) 6 months after myocardial infarction.

PrPC (or PrPc, or PRNP for Prion Protein) is a 253 amino acids protein presenting one disulfide bond, a molecular mass of 35-36 kDa and a mainly alpha-helical structure. PrPC is particularly abundant in brain, but has been shown to be also expressed in muscle, heart, lymphoid tissues, kidney, digestive tract, skin, mammary gland and endothelia.

The gene coding for the PrPC protein is recorded as number 6521 on the Gene database of the NCBI (National Center for Biotechnology Information, U.S. National Library of Medicine 8600 Rockville Pike, Bethesda MD, 20894 USA).

Relevant accession numbers for the protein are NP_000302.1, NP_001073590.1, NP_001073591.1, NP_001073592.1, NP_898902.1 or NP_001258490.1 (this corresponding to a variant). Variants of the protein (due to alternative splicing) also exist. This protein is thus known in the art. Some tests exist to dose it, although it has never been proposed to dose it in the plasma, and to determine or predict LVR.

The quality of a test is generally determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the test, for different thresholds (from 0 to 1).

It is usually acknowledged that a ROC curve, the area under which has a value superior to 0.7, is a good predictive curve. The ROC curve has to be acknowledged as a curve allowing prediction of the quality of a test. It is best for the AUROC to be as closed as 1 as possible, this value describing a test which is 100 % specific and sensitive.

It is reminded that
(1) Sensitivity is the probability that the diagnosis is positive in individuals having the phenotype sought (detection of true positives): the test is positive if the patient is having the phenotype. The sensitivity is low when the number of false negatives is high. The sensitivity is calculated by the formula SE = (number of individuals having the phenotype in whom the sign is present)/(number of individuals having the phenotype in whom the sign is present + number of individuals having the phenotype in whom the sign is absent).
(2) Specificity is the probability that the diagnosis is negative in the individuals not having the phenotype sought (non-detection of true negatives): the test is negative if the patient is not having the phenotype. The specificity is low when the number of false positives is high. The specificity is calculated by the formula SP = (number of individuals not having the phenotype in whom the sign is absent)/(number of individuals not having the phenotype in whom the sign is absent + number of individuals not having the phenotype in whom the sign is present).
(3) Positive predictive value (PPV): is the probability of having the disease if the diagnostic test is positive (i.e. that the patient is not a false positive): the patient is having the phenotype if the test is positive. The positive predictive value is calculated by the formula PPV = (number of individuals having the phenotype in whom the sign is present)/(number of individuals having the phenotype in whom the sign is present + number of individuals not having the phenotype in whom the sign is present).
(4) Negative predictive value (NPV): is the probability of not having the disease if the diagnostic test is negative (that the patient is not a false negative): the patient is not having the phenotype if the test is negative. The negative predictive value is calculated by the formula NPV = (number of individuals not having the phenotype in whom the sign is absent)/(number of individuals not having the phenotype in whom the sign is absent + number of individuals having the phenotype in whom the sign is absent)

Generally, a diagnosis (or prognosis) method comprises
i. a step of gathering information from the patient
ii. a step of comparing said information with regards to thresholds
iii. a step of deducing, from the difference between the patient's information and the threshold, whether the patient has a specific disease, the stage of the patient's disease, or whether the patient's state will evolve to a given state.

It is clear that the specificity, sensitivity, PPV and NPV depend on the threshold that is chosen. A "lower" threshold will decrease the specificity of the test (more false-positive), whereas a "higher" threshold will decrease the sensitivity of the test (some true positive will not be detected).

It is also to be noted that the method herein disclosed is not "gold-standard" tests, in the sense that the output (value of the PrPC) isn't a definitive answer as to the state of the patient. In fact, indicating that the method "predicts" the LVR six-months post myocardial infarction is similar to indicating that the probability of LVR in a given patient is higher than 50% (i.e. that it is more likely that there will be LVR than not). However, in view of the fact that the method is not gold-standard but is using an indirect marker and is based on statistics, some patients will be false-positive in the test, and some patients may be false-negative. This is the reason why the specific experience of the physician in interpreting the result is of importance for making the prognosis and deciding which kind of follow up is to be made to be made for each patient.

Thus, depending on the specificity, sensitivity, positive predictive value and negative predictive value of the tests, for various thresholds, the physician must interpret the result from the clinical and general context to be able to reach a conclusion. These tests are of great interest in provided a help to the physician when investigating a clinical case.

The invention thus relates to a method for determining whether a subject having suffered myocardial infarction will have deleterious left ventricular remodeling (LVR) within six months, comprising:
a) Comparing the level of PrPc in the plasma of the subject to a predetermined threshold
wherein the subject will have LVR within six months after myocardial infarction if the level of PrPc is higher than the threshold.

In another embodiment, the invention relates to a method for determining whether a subject having suffered myocardial infarction has deleterious left ventricular remodeling (LVR) six months after myocardial infarction, comprising:
a) Comparing the level of PrPc in the plasma of the subject six months after myocardial infarction to a predetermined threshold,
wherein the subject has LVR if the level of PrPc is higher than the threshold.

In a further embodiment, the invention relate to the above methods further comprising determining the level of PrPc in the plasma of the subject prior to a).

Consequently, the invention relates to a method for determining whether a subject having suffered myocardial infarction will have deleterious left ventricular remodeling (LVR) within six months, comprising:
a0) Determining the level of PrPc in a sample from the subject six months after myocardial infarction
a) Comparing the level of PrPc to a predetermined threshold,
wherein the subject has LVR if the level of PrPc is higher than the threshold.

The invention also relates to a method for determining whether a subject having suffered myocardial infarction will have deleterious left ventricular remodeling (LVR) within six months, comprising:
a0) Determining the level of PrPC in a sample from the subject
a) Comparing the level of PrPc to a predetermined threshold,
wherein the subject will have LVR within six months after myocardial infarction if the level of PrPc is higher than the threshold.

In a preferred embodiment, the level (amount) of PrPC is measured by ELISA assay. Such ELISA kits are available in the art, and are sold in particular by Biocompare (San Francisco, USA) or AJ Roboscreen GmbH (Leipzig, Germany, as BetaPrion HUMAN ELISA) and distributed by Analytik Jena (lena, Germany).

The inventors have noticed that the level of PrPC in the blood of subjects who have LVR is higher than the level of PrPC in the blood of subjects who don't have LVR. Generally, the level of PrPC in the blood of subjects who don't have LVR is lower than 5 ng/ml. It is however preferred to use plasma or serum of the subjects dans the dosing values mentioned therein are well applicable for measurement of the PrPC in the subjects' plasma.

Using 5 ng/ml as a threshold makes it possible to obtain a Sensitivity of 93.6% and Specificity of 73.9%.

Alternatively, if one wishes to increase the specificity, a higher threshold can be used, such as 5.25 ng/ml, 5.5 ng/ml, 6 ng/ml, 6.5 ng/l, 7 ng/ml or 7.5 ng/ml.

Using a threshold of 7 ng/ml makes it possible to obtain a Senstivity of 81% and a Specificity of 92.5%.

The inventors have further determined that the level of PrPC, when measured after the occurrence of the myocardial infraction, is higher in patients who will ultimately (within 6 months) present LVR, than in patients who will not present it. Such dosing of the PrPC is preferably performed shortly (i.e. preferably within one week) after occurrence of the myocardial infraction, so that the treatment can be adapted as early as possible. Dosing PrPC from day 3 to day 5 post-infarction, in particular at day 4 (the PrPc level is determined four days after myocardial infarction) is perfectly suitable.

The same thresholds as indicated above can be used.

Choice of the threshold depends on whether the physician wishes to increase the specificity or the sensitivity.

The invention also relates to a method for treating a subject having suffered myocardial infarction, comprising performing the method as disclosed above, so to that the risk of having a deleterious LVR within six months of the myocardial infarction is determined and wherein beta-blockers and at least one of ACE inhibitors, ARBs, MRA and Angiontensin-Receptor-Neprilysin-Inhibitors, are administered to the subject, when the subject is determined at risk of having deleterious LVR (i.e. its PrPC value is higher than the selected threshold).

The invention also relates to beta-blockers and at least one compound selected from the group consisting of ACE inhibitors, ARB, MRA and Angiontensin-Receptor-Neprilysin-Inhibitors for use thereof for the treatment of a subject having suffered myocardial infraction, when the subject is determined at risk of having deleterious LVR.

Such use can be simultaneous, separate or sequential (spread out over time).

Preferably betablocker and at least two of the listed drugs are administered to the subject if determined at risk.

This makes it possible to treat patients at risk of LVR with appropriate drugs aiming at preventing the occurrence of LVR and avoid over-treatment of patients that are not at risk, thereby limiting the risks for drug interactions and the cost for the health system.

### DESCRIPTION OF THE FIGURES

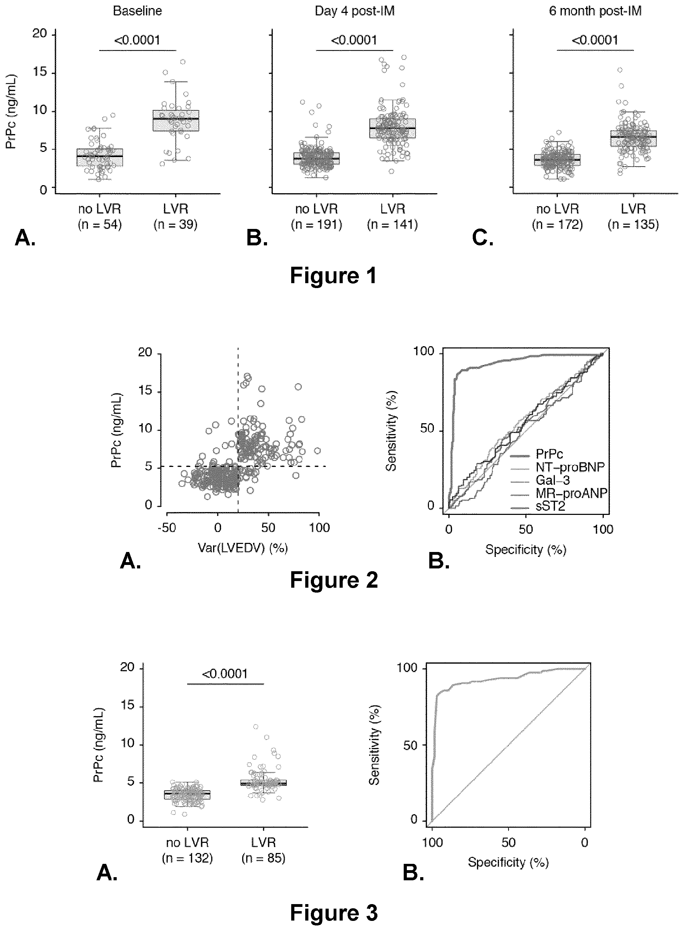

Figure 1: plasma measurements of PrPc in the PREGICA cohort. No LVR: no Left Ventricular Remodeling 6 month after myocardial infraction (MI). LVR: no Left Ventricular Remodeling 6 month after myocardial infraction. A. Measurements upon hospital entry. B. Measurements four days after occurrence of myocardial infraction. C. Measurements six months after occurrence of myocardial infraction.
Figure 2: A. distribution of plasma PrPc at day 4 post-MI against LVR considered as a continuous variable. Indicated as dashed lines are the PrPc and LVR values the discriminate the best non-remodellers and remodellers. B. ROC analysis of plasma PrPc (not aligned with the diagonal), NT-proBNP (N-terminus of brain natriuretic peptide), Gal-3 (Galectin-3), MR-proANP (Mid-regional pro atrial natriuretic peptide), and sST2 (soluble ST2 protein) measured at day 4 post-MI (all aligned with the diagonal).
Figure 3: A. plasma PrPc measured at day 4 post-MI in patients according to remodelling evaluated at 6 months after the MI. B. ROC analysis of plasma PrPc in REV2 measured at day 4 post-MI.

### EXAMPLES

### Example 1.

The PREGICA cohort (NCT01113268) enrolled patients with acute MI: cardiac remodelling was assessed by echocardiography at Day 4 and 6 months after MI, blood sampling was available at baseline, Day 4 and 6 month. LVR was calculated as the variation in left ventricular end-diastolic volume (LVEDV); patients with a variation in LVEDV > 20% were considered as remodellers. In those patients, we found the plasm PrPc was higher at baseline and Day 4 in those who will remodel by 6 month, when compared to those who will not. PrPc was still higher in remodeller patients at 6 month (once remodelling had already occurred), when compared to non-remodellers (figure 1).

At day 4 post-MI, plasma PrPc was highly predictive of future LVR in PREGICA patients (Figure 2 and Table 1) and outperformed standard cardiac biomarkers such as NT-proBNP, MR-proANP, Gal-3, and sST2. Therefore, measuring PrPc at 4 days post-MI would allow identifying patients at risk of LVR and adapt their management.

**Table 1: results from the ROC analysis to evaluate the ability of plasma PrPc to discriminate non-remodellers from remodellers. At baseline and day 4, results indicate the ability to predict the development of LVR at 6 months. At 6 months, results indicate the ability of plasma PrPc to identify non-remodellers from remodellers. NPV: negative predictive value, PPV: positive predictive value.**

| *Time point* | *AUC* | *Sensitivity* | *Specificity* | *NPV* | *PPV* |
|---|---|---|---|---|---|
| Baseline | 89.9% | 84.6% | 88.9% | 88.9% | 84.6% |
| Day 4 | 94% | 89.3% | 91.6% | 92.1% | 88.8% |
| 6 Months | 90.6% | 85.9% | 83.7% | 88.3% | 80.6% |
| REVE2 | 93.3% | 85.9% | 92.4% | 91.0% | 88.0% |

**Table 2: variation of the sensitivities and specificities to discriminate non-remodellers from remodellers for various plasma PrPc threshold at day 4 post MI.**

| *Criterion (ng*/*ml)* | *Sensitivity* | *95% Cl* | *Specificity* | *95% Cl* |
|---|---|---|---|---|
| >5.0 | 93.6 | 88.3 - 97.1 | 73.9 | 67.0 - 80.1 |
| >5.25 | 92.9 | 87.4 - 96.6 | 76.0 | 69.3 - 82.0 |
| >5.5 | 90.8 | 84.9 - 95.0 | 79.3 | 72.8 - 84.8 |
| >7.0 | 81.0 | 73.6 - 87.1 | 89.3 | 84.0 - 93.4 |
| >7.5 | 75.3 | 67.4 - 82.2 | 92.5 | 87.8 - 95.9 |

Besides its capacity to predict the development of LVR at 6 months when measured at day 4 post-MI, figure 1 shows that PrPC was also diagnostic of LVR at 6 months. Therefore, measuring PrPC at 6 +month would allow the patients with LVR without necessarily perform echocardiography or cardiac MRI.

The predictive value of PrPc measured at day 4 post-MI for LVR was confirmed in another cohort of patients, REVE2 (Principal Investigator: C. Bauters, Lille; figure 3 and table 1) in which patients share similar characteristics with those from PREGICA.

Altogether, it was found that measuring the dosage of PrPC is a better predictor than LVEF to predict remodeling at six months.

## Claims

1. A method for determining whether a subject having suffered myocardial infarction will have deleterious left ventricular remodeling (LVR) within six months, comprising:
a) Comparing the level of PrPc in the plasma of the subject to a predetermined threshold
wherein the subject will have LVR within six months after myocardial infarction if the level of PrPc is higher than the threshold.

2. A method for determining whether a subject having suffered myocardial infarction has deleterious left ventricular remodeling (LVR) six months after myocardial infarction, comprising:
a) Comparing the level of PrPc in the plasma of the subject six months after myocardial infarction to a predetermined threshold,
wherein the subject has LVR if the level of PrPc is higher than the threshold.

3. The method of claim 1 or 2, further comprising determining the level of PrPc in the plasma of the subject prior to a).

4. The method of any one of claims 1 to 3, wherein the threshold is comprised between 5 ng/ml and 7.5 ng/ml.

5. The method of claim 4, wherein the threshold is 5.5 ng/ml.

6. The method of claim 4, wherein the threshold is 7 ng/ml.

7. The method of any one of claims 1, and 3 to 6, wherein the PrPc level has been determined four days after myocardial infarction.

8. Beta-blockers and at least one compound selected from the group consisting of ACE inhibitors, ARB, MRA and Angiontensin-Receptor-Neprilysin-Inhibitors for use thereof for the treatment of a subject having suffered myocardial infraction, when the subject is determined at risk of having deleterious LVR by a method according to any one of claims 1, and 3 to 7.
